# EUROPÄISCHE PATENTSCHRIFT

(11) **EP 4 198 547 B1**
(45) Veröffentlichungstag und Bekanntmachung des Hinweises auf die Patenterteilung: **06.11.2024**
(21) Anmeldenummer: 21215696.2
(22) Anmeldetag: 17.12.2021
(51) Int. Cl.: G01R 33/48, G01R 33/56, A61B 5/00, A61B 34/10, G06T 7/00, G06T 7/11

(54) **PLANUNGSEINHEIT UND VERFAHREN ZU EINER VORBEREITUNG EINES ÄSTHETISCHEN OPERATIVEN EINGRIFFES**
PLANNING UNIT AND METHOD FOR PREPARING AN AESTHETIC SURGICAL PROCEDURE
UNITÉ DE PLANIFICATION ET PROCÉDÉ DE PRÉPARATION D'UNE INTERVENTION CHIRURGICALE ESTHÉTIQUE

(43) Veröffentlichungstag der Anmeldung: 21.06.2023
(73) Patentinhaber: Siemens Healthineers AG, 91301 Forchheim (DE)
(72) Erfinder: Zeller, Mario, 91054 Erlangen (DE)
(74) Vertreter: Siemens Healthineers Patent Attorneys

(56) Entgegenhaltungen:
- US-B2- 8 275 442
- SURESH ANAND SADANANTHAN ET AL: "Automated segmentation of visceral and subcutaneous (deep and superficial) adipose tissues in normal and overweight men : Automated Segmentation of Adipose Tissue", JOURNAL OF MAGNETIC RESONANCE IMAGING, vol. 41, no. 4, 7 May 2014 (2014-05-07), US, pages 924 - 934, XP055638832, ISSN: 1053-1807, DOI: 10.1002/jmri.24655
- MADDALO MICHELE ET AL: "Validation of a free software for unsupervised assessment of abdominal fat in MRI", PHYSICA MEDICA, ACTA MEDICA EDIZIONI E CONGRESSI, ROME, IT, vol. 37, 10 April 2017 (2017-04-10), pages 24 - 31, XP085033753, ISSN: 1120-1797, DOI: 10.1016/J.EJMP.2017.04.002
- HOAD C.L. ET AL.: "A semi-automtic method to segment visceral, subcutaneous and total fat in the abdoment from MRI data", PROCEEDINGS OF THE INTERNATIONAL SOCIETY FOR MAGNETIC RESONANCE IN MEDICINE, JOINT ANNUAL MEETING ISMRM-ESMRMB, no. 4310, 28 April 2014 (2014-04-28), pages 4310, XP040671083
- DANILLA STEFAN ET AL: "High-Definition Liposculpture: What are the Complications and How to Manage Them?", AESTHETIC PLASTIC SURGERY, SPRINGER VERLAG, NEW YORK, NY, US, vol. 44, no. 2, 20 August 2019 (2019-08-20), pages 411 - 418, XP037064090, ISSN: 0364-216X, [retrieved on 20190820], DOI: 10.1007/S00266-019-01475-6

## Beschreibung

Die Erfindung betrifft ein Verfahren, eine Planungseinheit, ein Computerprogrammprodukt sowie einen elektronisch lesbaren Datenträger zu einer Vorbereitung eines ästhetischen operativen Eingriffes.

Die ästhetische Medizin ist ein Wachstumsmarkt und eine häufig beanspruchte Behandlungsform ist die Körperfettentfernung via Liposuktion oder Lipolyse. Die Planung, Simulation und die Visualisierung des Ergebnisses des Eingriffs können softwarebasiert erfolgen, wobei Daten einer 3D-Kamera verwendet werden. Die Visualisierung solcher Daten ist rein illustrativ.

Der Artikel Sadananthan S.A. et al., "Automated segmentation of visceral and subcutaneous (deep and superficial)adipose tissues in normal and overweigth men: Automated Segmentation of Adipose Tissue", JMRI, Bd. 41, Nr. 4, 2014, S. 924-934, beschreibt ein Verfahren zur Segmentierung von Lipid-MR-Daten zur Abgrenzung von Fettgewebe.

Das U.S. Patent 8 275 442 B2 beschreibt Verfahren zur Identifizierung von zu entfernendem Fettgewebe, wobei insbesondere MRT zum Einsatz kommen kann.

Der Erfindung liegt die Aufgabe zugrunde, ein besonders präzises Verfahren für eine visuell leicht verständliche Planung, Durchführung und Simulation eines Ergebnisses eines anstehenden ästhetischen operativen Eingriffes anzugeben. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zu einer Vorbereitung eines ästhetischen operativen Eingriffes hinsichtlich eines Untersuchungsbereiches eines Untersuchungsobjektes sieht die folgenden Verfahrensschritte vor:
- Bereitstellung von präoperativen MR-Bilddaten des Untersuchungsbereiches umfassend Lipid-MR-Daten aufweisend einen Lipidkontrast und umfassend Wasser-MR-Daten aufweisend einen Wasserkontrast,
- Segmentierung der Lipid-MR-Daten zur Abgrenzung von Fettgewebe von umgebendem Gewebe mittels einer Segmentierungseinheit,
- Erstellung einer Lipidkarte des Untersuchungsobjektes basierend auf den segmentierten Lipid-MR-Daten mittels einer Extraktionseinheit,
- Bestimmung einer Lipiddichtkarte umfassend einen relativen Fettanteil basierend auf den Wasser-MR-Daten und den Lipid-MR-Daten mittels einer Betimmungseinheit,
- Bereitstellung einer Referenzkarte für Fettgewebe repräsentativ für einen Idealzustand, wobei der Idealzustand abhängig von individuellen Daten des Untersuchungsobjektes ist,
- Bereitstellung einer weiteren Referenzkarte für eine Lipiddichte repräsentativ für den Idealzustand,
- Ermittlung eines Vergleichsergebnisses umfassend eine Differenz zwischen der Lipidkarte und der Referenzkarte und eine Differenz zwischen der Lipiddichtkarte und der weiteren Referenzkarte mittels einer Differenzierungseinheit,
- Bestimmung zumindest eines Teilbereichs der segmentierten Lipid-MR-Daten basierend auf dem Vergleichsergebnis, welcher Teilbereich einem zu entfernenden Gewebe entspricht, umfassend eine Prüfung des Teilbereiches hinsichtlich einer pathologischen und/oder physiologischen Relevanz mittels der Bestimmungseinheit,
- Bereitstellung des Vergleichsergebnisses.

Ein ästhetischer operativer Eingriff ist typischerweise ein ästhetisch begründeter chirurgischer Eingriff bei einem Patienten, insbesondere bei einem Untersuchungsobjekt, wobei vom Untersuchungsbereich Gewebe entfernt und/oder umgeformt wird. Der Untersuchungsbereich kann den gesamten Körper des Untersuchungsobjektes und/oder einen Teilbereich des Untersuchungsobjektes, insbesondere ein äußerlich sichtbares Körperteil umfassen.

Präoperativen MR-Bilddaten umfassen MR-Bilddaten, welche mittels eines Magnetresonanzgerätes von dem Untersuchungsbereich des Untersuchungsobjektes vor Durchführung eines ästhetischen operativen Eingriffes erfasst werden. Die Bereitstellung der präoperativen MR-Bilddaten kann eine Akquisition der präoperativen MR-Bilddaten durch Ansteuerung eines Magnetresonanzgerätes gemäß einer MR-Steuerungssequenz umfassen. Eine MR-Steuerungssequenz umfasst typischerweise eine Folge von Hochfrequenz-Pulsen, beispielsweise Anregungspulsen und Refokussierungspulsen, sowie passend dazu koordiniert auszusendenden Gradientenpulsen in verschiedenen Gradientenachsen entlang verschiedener Raumrichtungen. Zeitlich passend hierzu werden Auslesefenster gesetzt, welche die Zeiträume vorgeben, in denen die vom Untersuchungsbereich induzierten Magnetresonanz-Signale erfasst werden. Erfasste Magnetresonanz-Signale können zu MR-Bilddaten rekonstruiert werden. MR-Bilddaten bilden typischerweise den Untersuchungsbereich in zueinander parallelen zweidimensionalen Schichten und/oder dreidimensional ab. Die Bereitstellung der präoperativen MR-Bilddaten kann eine Datenübertragung umfassen, wobei die präoperativen MR-Bilddaten bereits als Datei vorliegen.

Lipid-MR-Daten aufweisend einen Lipidkontrast sind typischerweise MR-Bilddaten, welche vorzugsweise dadurch gekennzeichnet sind, dass eine Signalintensität von Gewebe dominiert von Lipiden zumindest doppelt so groß oder höchstens halb so groß ist wie eine Signalintensität von Gewebe dominiert von Wasser und/oder Muskulatur. Lipid-MR-Daten aufweisend einen Lipidkontrast sind vorzugsweise dadurch gekennzeichnet, dass Gewebe umfassend Lipide einen starken Kontrast zu Gewebe umfassend Wasser und/oder Muskulatur aufweist. Lipid-MR-Daten aufweisend einen Lipidkontrast sind vorzugsweise dadurch gekennzeichnet, dass Gewebe umfassend Lipide einen stärkeren Kontrast, insbesondere größeren Signalunterschied, zu Muskulatur aufweist als Sehnen und/oder Knochen zu Muskulatur.

Die Segmentierung der Lipid-MR-Daten hinsichtlich Fettgewebe umfasst eine Abgrenzung, insbesondere eine dreidimensionale Abgrenzung, von Fettgewebe vom umgebenden Gewebe. Fettgewebe ist typischerweise ein von Lipiden dominiertes Gewebe. Insbesondere kann die Segmentierung der Lipid-MR-Daten eine Volumetrie des Fettgewebes umfassen.

Die Lipidkarte umfasst eine Information hinsichtlich einer räumlichen Verteilung des Fettgewebes. Die Verwendung von präoperativen MR-Bilddaten des Untersuchungsobjektes ermöglicht insbesondere eine Bestimmung der tatsächlichen Verteilung des Fettgewebes, welche die Lipidkarte typischerweise wiedergibt.

Referenzkarte für Fettgewebe repräsentativ für einen Idealzustand umfasst typischerweise eine Lipidkarte eines Referenzobjektes, welches Referenzobjekt einem Idealzustand entspricht. Ein Idealzustand entsprich typischerweise einem subjektiven und/oder von einer Mehrheit anerkannten Schönheitsideal, insbesondere hinsichtlich Figur und/oder hinsichtlich äußerer Form eines Körperteils. Die Referenzkarte umfasst vorzugsweise eine Lipidkarte eines Referenzobjektes für den Untersuchungsbereich.

Das Vergleichsergebnis umfasst insbesondere Abweichungen des Untersuchungsobjektes vom Idealzustand für Fettgewebe, insbesondere für den Untersuchungsbereich. Das Vergleichsergebnis kann als Vergleichskarte ausgebildet sein und/oder die Abweichungen des Untersuchungsobjektes vom Idealzustand räumlich aufgelöst umfassen. Die Bereitstellung des Vergleichsergebnisses kann eine zur Verfügungstellung und/oder eine Übertragung einer Datei umfassend das Vergleichsergebnis sein.

Die Ermittlung eines Vergleichsergebnisses, darstellend einen Unterschied zwischen dem Fettgewebe des Untersuchungsobjektes und einem Fettgewebe repräsentativ für einen Idealzustand, kann aufgrund der Verwendung von MR-Bilddaten, insbesondere von Lipid-MR-Daten, besonders genau erfolgen. Insbesondere die Kenntnis der tatsächlichen Verteilung des Fettgewebes in Form der Lipidkarte ermöglicht eine präzise Visualisierung des präoperativen Zustandes, eine realistische Planung eines operativen Eingriffes und auch eine Simulation eines realistischen Ergebnisses eines anstehenden ästhetischen operativen Eingriffes umfassend eine Entfernung von Fettgewebe.

Eine Ausführungsform des Verfahrens sieht vor, dass die Bereitstellung des Vergleichsergebnisses eine farbliche Kodierung des Vergleichsergebnisses und Visualisierung von farblich kodierten Differenzen umfasst.

Die Abweichungen des Untersuchungsobjektes vom Idealzustand für Fettgewebe und/oder eine Lipidklasse und/oder eine Lipiddichte können farblich kodiert werden und einem medizinischen Personal, insbesondere einem Chirurgen, und/oder dem Patienten angezeigt werden. Diese Visualisierung verdeutlicht typischerweise Fettgewebe, welches im Rahmen eines chirurgischen Eingriffes entfernbar ist. Insbesondere kann dem Untersuchungsobjekt dessen Abweichungen von der Referenzkarte und mögliche Änderungen gut dargestellt werden. Ebenso können derartige Darstellungen bei der Patientenaufklärung unterstützen.

Eine Ausführungsform des Verfahrens sieht vor, dass der Idealzustand abhängig von individuellen Daten des Untersuchungsobjektes ist. Die Bereitstellung der Referenzkarte kann insbesondere eine Selektion eines für das Untersuchungsobjekt erstrebenswerten Idealzustandes umfassen. Die Referenzkarte und/oder der Idealzustand sind vorzugsweise abhängig von zumindest einer der folgenden Eigenschaften des Untersuchungsobjektes:
- Körpergröße
- Alter
- Geschlecht
- Trainingszustand
- Gewicht
- Körperform
- Kulturform
- subjektivem Schönheitsideal des Untersuchungsobjektes.

Diese Ausführungsform ermöglicht eine Verwendung einer für das Untersuchungsobjekt individuell passenden Referenzkarte. Dies ermöglicht eine Erzeugung eines gesunden, individuellen Vergleichsergebnisses.

Das Verfahren umfasst eine Bestimmung zumindest eines Teilbereiches der segmentierten Lipid-MR-Daten basierend auf dem Vergleichsergebnis und/oder der Lipidkarte, welcher Teilbereich einem zu entfernenden Gewebe entspricht. Dies ermöglicht eine Planung des operativen Eingriffes durch Selektion des zu entfernenden Fettgewebes. Der Teilbereich ist vorzugsweise eine Teilmenge des Fettgewebes.

Eine Ausführungsform des Verfahrens sieht vor, dass die Bestimmung des zumindest einen Teilbereiches der segmentierten Lipid-MR-Daten eine Auswahl eines Körperbereiches des Untersuchungsobjektes und/oder eine Vorgabe einer Menge eines zu entfernenden Gewebes umfasst. Der Körperbereich kann beispielsweise eine Extremität und/oder das Abdomen und/oder den Gesäßbereich umfassen. Dieser Körperbereich entspricht typischerweise dem Teilbereich des Untersuchungsobjektes, welcher durch den operativen Eingriff verändert werden soll. Die Vorgabe der Menge kann eine Vorgabe eines Volumens sein. Vorzugsweise wird der Teilbereich automatisiert basierend auf dem vorgegebenen Körperbereich und/oder der Menge derart bestimmt, dass der Teilbereich im Umfeld des Körperbereiches die Menge umfasst, der Teilbereich physiologisch redundant und entfernbar ist, und der Untersuchungsbereich abzüglich des Teilbereichs einem natürlichen Erscheinungsbild entspricht. Diese Ausführungsform ermöglicht eine Simulation verschiedener operativer Eingriffe und damit eine besonders gute Planung.

Das erfindungsgemässe Verfahren sieht vor, dass die Bestimmung des zumindest einen Teilbereiches eine Prüfung des Teilbereiches hinsichtlich einer pathologischen und/oder physiologischen Relevanz, wie beispielsweise Gefäße, umfasst.

Dies stellt sicher, dass funktionale Strukturen vom Teilbereich des zu entfernenden Gewebes ausgeschlossen werden. Dadurch kann das Risiko des operativen Eingriffes reduziert werden.

Eine Ausführungsform des Verfahrens umfasst zusätzlich eine Erzeugung simulierter MR-Bilddaten durch Entfernung des Teilbereiches aus den präoperativen MR-Bilddaten.

Die Erzeugung simulierter MR-Bilddaten umfasst vorzugsweise eine virtuelle Entfernung von Fettgewebe. Dies kann dem Untersuchungsobjekt und dem behandelnden medizinischem Personal als Entscheidungshilfe über das Ausmaß eines möglichen operativen Eingriffes dienen.

Eine Ausführungsform des Verfahrens umfasst zusätzlich eine perspektivische Visualisierung einer Oberfläche der simulierten MR-Bilddaten. Gemäß dieser Ausführungsform umfasst die Erzeugung simulierter MR-Bilddaten vorzugsweise eine virtuelle Entfernung von Fettgewebe und ein entsprechendes Schrumpfen der Körperoberfläche um den Untersuchungsbereich abzüglich des Teilbereiches. Dies lässt sich insbesondere mit Verfahren wie "cinematic rendering" besonders detailgetreu und realistisch dreidimensional darstellen.

Eine Ausführungsform des Verfahrens umfasst zusätzlich eine dreidimensionale photographische Aufnahme der Körperoberfläche zumindest eines Ausschnittes des Untersuchungsobjektes, welche bei der perspektivischen Visualisierung der Oberfläche der simulierten MR-Bilddaten berücksichtigt wird. Die dreidimensionale photographische Aufnahme der Körperoberfläche umfasst vorzugsweise eine präoperative Aufnahme des Untersuchungsbereiches und/oder des Körperbereiches mittels 3D-Kamera. Die perspektivische Visualisierung umfasst vorzugsweise eine Fusionierung der dreidimensionalen photographischen Aufnahme und der simulierten MR-Bilddaten. Dies ermöglicht eine Kombination der geometrietreuen quantitativen Werte der simulierten MR-Bilddaten mit dem Fotorealismus der photographischen Aufnahme.

Eine Ausführungsform des Verfahrens sieht vor, dass die präoperative MR-Bilddaten als drei-dimensionale MR-Bilddaten ausgebildet sind.

Eine Ausführungsform des Verfahrens sieht vor, dass die Bereitstellung der präoperativen MR-Bilddaten deren Akquisition unter Verwendung einer DIXON-MR-Steuerungssequenz, insbesondere einer VIBE-DIXON-MR-Steuerungssequenz, umfasst. Eine DIXON-MR-Steuerungssequenz ist typischerweise dazu ausgebildet, gleichzeitig MR-Bilddaten verschiedener Kontraste, insbesondere eines Lipidkontrasts und eines Wasserkontrastes, zu erzeugen. Die Akquisition derartiger präoperativer MR-Bilddaten kann also besonders effizient und kostengünstig erfolgen. Insbesondere von einem Abdomen können mittels einer VIBE-DIXON-MR-Steuerungssequenz innerhalb weniger Sekunden dreidimensionale MR-Bilddaten erfasst werden. Dies reduziert Bewegungsartefakte während der Akquisition und ermöglicht zugleich eine besonders genaue Planung eines operativen Eingriffes basierend auf isotropen MR-Bilddaten.

Das erfindungsgemässe Verfahren sieht vor, dass die präoperativen MR-Bilddaten zusätzlich Wasser-MR-Daten aufweisend einen Wasserkontrast umfassen, das Verfahren zusätzlich
- eine Bestimmung einer Lipiddichtkarte umfassend einen relativen Fettanteil basierend auf den Wasser-MR-Daten und den Lipid-MR-Daten, und
- eine Bereitstellung einer weiteren Referenzkarte für eine Lipiddichte repräsentativ für einen Idealzustand, vorsieht,
und die Ermittlung des Vergleichsergebnisses eine Differenz zwischen der Lipiddichtkarte und der weiteren Referenzkarte umfasst.

Wasser-MR-Daten aufweisend einen Wasserkontrast sind typischerweise MR-Bilddaten, welche vorzugsweise dadurch gekennzeichnet sind, dass eine Signalintensität von Gewebe dominiert von Wasser und/oder Muskulatur zumindest doppelt so groß oder höchstens halb so groß ist wie eine Signalintensität von Gewebe dominiert von Fett. Wasser-MR-Daten aufweisend einen Wasserkontrast sind vorzugsweise dadurch gekennzeichnet, dass Gewebe umfassend Wasser und/oder Muskulatur einen starken Kontrast zu Gewebe umfassend Fett aufweist. Wasser-MR-Daten aufweisend einen Wasserkontrast sind vorzugsweise dadurch gekennzeichnet, dass Gewebe umfassend Wasser und/oder Muskulatur einen stärkeren Kontrast, insbesondere größeren Signalunterschied, zu Fett aufweist als Sehnen und/oder Knochen zu Wasser und/oder Muskulatur.

Die Lipiddichtkarte umfassend einen relativen Fettanteil kann durch voxelweise und/oder punktweise Division der Lipid-MR-Daten durch die Wasser-MR-Daten erzeugt werden und/oder kann eine räumlich aufgelöste Lipiddichte und/oder einen räumlich aufgelösten Fettanteil umfassen. Das Vergleichsergebnis gemäß dieser Ausführungsform berücksichtigt einen Fettanteil, wodurch eine individuellere Aussage zur tatsächlichen Lipidverteilung im Untersuchungsobjekt möglich ist. Eine Berücksichtigung der Lipiddichte bei der Planung des operativen Eingriffes, insbesondere bei der Bestimmung des Teilbereiches, ist zuverlässiger und reduziert Risiken.

Eine Ausführungsform des Verfahrens umfasst zusätzlich eine
- Subsegmentierung der segmentierten Lipid-MR-Daten gemäß zumindest zwei Lipidklassen,
- Klassifizierung der subsegmentierten Lipid-MR-Daten gemäß den zumindest zwei Lipidklassen,
wobei bei Erstellung der Lipidkarte eine erste Lipidklasse der zumindest zwei Lipidklassen ausgeschlossen wird und/oder die Lipidkarte eine Information hinsichtlich der Klassifizierung umfasst.

Die Subsegmentierung erfolgt vorzugsweise unter Berücksichtigung der räumlichen Position des Untersuchungsbereiches und/oder der Gewebestruktur der segmentierten Lipid-MR-Daten. Die erste Lipidklasse der zumindest zwei Lipidklassen umfasst typischerweise viszerales Fett. Eine zweite Lipidklasse der zumindest zwei Lipidklassen umfasst typischerweise subkutanes Fett. Diese Ausführungsform sieht demnach vor, dass eine Lipidklasse nicht als Lipid im Sinne von zu entfernendem Fettgewebe erfasst und/oder dargestellt wird. Die erste Lipidklasse wird vorzugsweise bei der Wahl des Teilbereiches ausgeschlossen. Dies erhöht die Genauigkeit der Planung und der Simulation des operativen Eingriffes, da viszerales Fett typischerweise nicht entfernt werden kann.

Eine Ausführungsform des Verfahrens umfasst zusätzlich eine Bereitstellung postoperativer MR-Bilddaten des Untersuchungsbereiches und einen Vergleich der postoperativen MR-Bilddaten mit den präoperativen MR-Bilddaten und/oder der Referenzkarte und/oder den simulierten MR-Bilddaten.

Die postoperativen MR-Bilddaten unterscheiden sich von den präoperativen MR-Bilddaten durch den Zeitpunkt ihrer Akquisition. Die postoperativen MR-Bilddaten sind dadurch gekennzeichnet, dass sie nach der Akquisition der präoperativen MR-Bilddaten und nach der Durchführung des anhand der präoperativen MR-Bilddaten geplanten operativen Eingriffes akquiriert wurden. Diese Ausführungsform ermöglicht eine Visualisierung des Erfolges für den Patienten, insbesondere eine Verlaufskontrolle. Zudem kann ausgehend von diesem Vergleich eine Folgebehandlung geplant werden.

Eine Ausführungsform des Verfahrens umfasst zusätzlich
- ein Erfassen einer Position eines Interventionsgerätes ausgebildet zum Entfernen von Fettgewebe während eines interventionellen Verfahrens zur Entfernung von Fettgewebe und
- eine Visualisierung der Position in Überlagerung mit den präoperativen MR-Bilddaten.

Das interventionelle Verfahren zur Entfernung von Fettgewebe unter Verwendung des Interventionsgerätes erfolgt vorzugsweise im Rahmen des operativen Eingriffes. Das Interventionsgerät kann beispielsweise als Nadel, Liposuktionsgerät, oder Ultraschallgerät ausgebildet sein. Das Erfassen der Position kann beispielsweise mittels optischer Verfahren und/oder Lageerkennung des Interventionsgerätes erfolgen. Das Erfassen der Position erfolgt vorzugsweise in Echtzeit. Die Visualisierung der Position in Überlagerung mit den präoperativen MR-Bilddaten ermöglicht typischerweise eine besonders präzise Steuerung des Interventionsgerätes und ein exaktes Entfernen des Teilbereiches, insbesondere des wie geplant zu entfernenden Fettgewebes. Die Visualisierung der Position in Überlagerung mit den präoperativen MR-Bilddaten unterstützt demnach den operativen Eingriff durch verbesserte Orientierung.

Des Weiteren geht die Erfindung aus von einer Planungseinheit gemäss Anspruch 9, mit einem Eingang, einer Segmentierungseinheit, einer Extraktionseinheit, einer Differenzierungseinheit und einem Ausgang. Die Planungseinheit ist dazu ausgebildet, ein erfindungsgemäßes Verfahren zu einer Vorbereitung eines ästhetischen operativen Eingriffes auszuführen. Über den Eingang können der Planungseinheit präoperative MR-Bilddaten, eine Referenzkarte für Fettgewebe repräsentativ für einen Idealzustand und/oder postoperative MR-Bilddaten bereitgestellt werden. Weitere, im Verfahren benötigte Funktionen, Algorithmen oder Parameter können der Planungseinheit über den Eingang bereitgestellt werden.

Das Vergleichsergebnis und/oder die simulierten MR-Bilddaten und/oder die perspektivische Visualisierung und/oder weitere Ergebnisse einer Ausführungsform des erfindungsgemäßen Verfahrens können über den Ausgang bereitgestellt werden.

Die Segmentierungseinheit ist ausgebildet zu einer Segmentierung der Lipid-MR-Daten hinsichtlich Fettgewebe. Die Segmentierungseinheit kann zu einer Subsegmentierung der segmentierten Lipid-MR-Daten gemäß zumindest zwei Lipidklassen und/oder zu einer Klassifizierung der subsegmentierten Lipid-MR-Daten gemäß den zumindest zwei Lipidklassen ausgebildet sein. Die Extraktionseinheit ist ausgebildet zu einer Erstellung einer Lipidkarte des Untersuchungsobjektes basierend auf den segmentierten Lipid-MR-Daten. Die Differenzierungseinheit ist ausgebildet zu einer Ermittlung eines Vergleichsergebnisses umfassend eine Differenz zwischen der Lipidkarte und der Referenzkarte und/oder eine Differenz zwischen der Lipiddichtkarte und einer weiteren Referenzkarte. Die Differenzierungseinheit kann zu einem Vergleich der postoperativen MR-Bilddaten mit den präoperativen MR-Bilddaten und/oder der Referenzkarte und/oder den simulierten MR-Bilddaten ausgebildet sein.

Die Planungseinheit kann zusätzlich eine Bestimmungseinheit umfassen, welche Bestimmungseinheit zu einer Bestimmung zumindest eines Teilbereiches der segmentierten Lipid-MR-Daten basierend auf dem Vergleichsergebnis und/oder der Lipidkarte ausgebildet ist. Die Bestimmungseinheit ist zur Bestimmung einer Lipiddichtkarte umfassend einen relativen Fettanteil basierend auf den Wasser-MR-Daten und den Lipid-MR-Daten und einer Prüfung des Teilbereiches hinsichtlich einer pathologischen und/oder physiologischen Relevanz ausgebildet. Die Bestimmungseinheit kann zu
- einem Erfassen einer Position eines Interventionsgerätes ausgebildet zum Entfernen von Fettgewebe während eines interventionellen Verfahrens zur Entfernung von Fettgewebe und
- einer Visualisierung der Position in Überlagerung mit den präoperativen MR-Bilddaten ausgebildet sein.

Die Planungseinheit kann zusätzlich eine Simulationseinheit umfassen, welche Simulationseinheit zu einer Erzeugung simulierter MR-Bilddaten durch Entfernung des Teilbereiches aus den präoperativen MR-Bilddaten ausgebildet ist. Die Planungseinheit kann zusätzlich eine Sensoreinheit umfassen, welche Sensoreinheit zu einer dreidimensionalen photographischen Aufnahme der Körperoberfläche zumindest eines Ausschnittes des Untersuchungsobjektes ausgebildet ist.

Die Planungseinheit kann in ein Magnetresonanzgerät integriert sein, welches Magnetresonanzgerät zu einer Akquisition der präoperativen MR-Bilddaten unter Verwendung einer DIXON-MR-Steuerungssequenz, insbesondere einer VIBE-DIXON-MR-Steuerungssequenz, und/oder zu einer Akquisition postoperativer MR-Bilddaten des Untersuchungsbereiches ausgebildet ist. Die Planungseinheit kann mit einem solchen Magnetresonanzgerät verbunden sein. Die Planungseinheit kann auch separat von dem Magnetresonanzgerät installiert sein. Ausführungsformen der erfindungsgemäßen Planungseinheit sind analog zu den Ausführungsformen des erfindungsgemäßen Verfahrens ausgebildet. Die Planungseinheit kann weitere Steuerungskomponenten aufweisen, welche zum Ausführen eines erfindungsgemäßen Verfahrens nötig und/oder vorteilhaft sind. Auch kann die Planungseinheit dazu ausgebildet sein, Steuerungssignale zu senden und/oder Steuerungssignale zu empfangen und/oder zu verarbeiten, um ein erfindungsgemäßes Verfahren auszuführen. Auf einer Speichereinheit der Planungseinheit können Computerprogramme und weitere Software gespeichert sein, mittels derer die Prozessoreinheit der Planungseinheit einen Verfahrensablauf eines erfindungsgemäßen Verfahrens automatisch steuert und/oder ausführt.

Ein erfindungsgemäßes Computerprogrammprodukt ist direkt in einer Speichereinheit einer programmierbaren Planungseinheit ladbar und weist Programmcode-Mittel auf, um ein erfindungsgemäßes Verfahren auszuführen, wenn das Computerprogrammprodukt in der Planungseinheit ausgeführt wird. Dadurch kann das erfindungsgemäße Verfahren schnell, identisch wiederholbar und robust ausgeführt werden. Das Computerprogrammprodukt ist so konfiguriert, dass es mittels der Planungseinheit die erfindungsgemäßen Verfahrensschritte ausführen kann. Die Planungseinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem elektronisch lesbaren Medium gespeichert oder auf einem Netzwerk oder Server hinterlegt, von wo es in den Prozessor einer lokalen Recheneinheit geladen werden kann, der mit der Planungseinheit direkt verbunden oder als Teil der Planungseinheit ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit einer Planungseinheit ein erfindungsgemäßes Verfahren durchführen. Beispiele für elektronisch lesbare Datenträger sind eine DVD, ein Magnetband oder einen USB-Stick, auf welchem elektronisch lesbare Steuerinformationen, insbesondere Software, gespeichert ist. Wenn diese Steuerinformationen (Software) von dem Datenträger gelesen und in eine Steuerungseinheit und/oder Planungseinheit gespeichert werden, können alle erfindungsgemäßen Ausführungsformen der vorab beschriebenen Verfahren durchgeführt werden.

Des Weiteren geht die Erfindung aus von einem elektronisch lesbaren Datenträger, auf dem ein Programm hinterlegt ist, das zu einer Ausführung eines Verfahrens zu einer Vorbereitung eines ästhetischen operativen Eingriffes, vorgesehen ist.

Die Vorteile der erfindungsgemäßen Planungseinheit, des erfindungsgemäßen Computerprogrammprodukts und des erfindungsgemäßen elektronisch lesbaren Datenträgers entsprechen im Wesentlichen den Vorteilen des erfindungsgemäßen Verfahrens zu einer Vorbereitung eines ästhetischen operativen Eingriffes, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: ein Ablaufdiagramm einer ersten Ausführungsform eines erfindungsgemäßen Verfahrens,
- Fig. 2: ein Ablaufdiagramm einer zweiten Ausführungsform eines erfindungsgemäßen Verfahrens,
- Fig. 3: ein Ablaufdiagramm einer dritten Ausführungsform eines erfindungsgemäßen Verfahrens,
- Fig. 4: ein Ablaufdiagramm einer vierten Ausführungsform eines erfindungsgemäßen Verfahrens,
- Fig. 5: ein Ablaufdiagramm einer fünften Ausführungsform eines erfindungsgemäßen Verfahrens,
- Fig. 6: ein Ablaufdiagramm einer sechsten Ausführungsform eines erfindungsgemäßen Verfahrens,
- Fig. 7: eine erfindungsgemäße Planungseinheit in einer schematischen Darstellung.

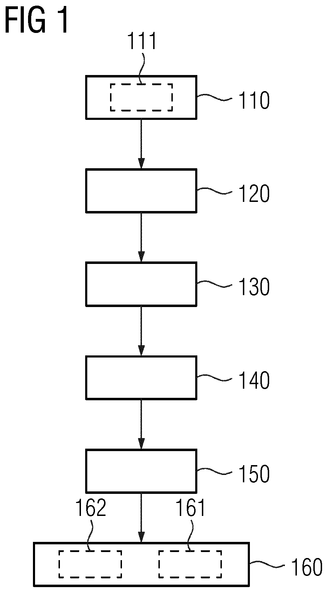

Figur 1 zeigt ein Ablaufdiagramm einer ersten Ausführungsform eines erfindungsgemäßen Verfahrens zu einer Vorbereitung eines ästhetischen operativen Eingriffes hinsichtlich eines Untersuchungsbereiches eines Untersuchungsobjektes, welches mit Verfahrensschritt 110, der Bereitstellung von präoperativen MR-Bilddaten des Untersuchungsbereiches umfassend Lipid-MR-Daten aufweisend einen Lipidkontrast, beginnt. Im folgenden Verfahrensschritt 120 erfolgt die Segmentierung der Lipid-MR-Daten hinsichtlich Fettgewebe. Verfahrensschritt 130 sieht die Erstellung einer Lipidkarte des Untersuchungsobjektes basierend auf den segmentierten Lipid-MR-Daten vor. Verfahrensschritt 140 umfasst eine Bereitstellung einer Referenzkarte für Fettgewebe repräsentativ für einen Idealzustand. Verfahrensschritt 140 kann auch simultan mit Verfahrensschritt 110 und/oder zu einem beliebigen Zeitpunkt vor Verfahrensschritt 150 umfassend eine Ermittlung eines Vergleichsergebnisses umfassend eine Differenz zwischen der Lipidkarte und der Referenzkarte, erfolgen. Verfahrensschritt 160 umfasst eine Bereitstellung des Vergleichsergebnisses.

Verfahrensschritt 160 kann optional mit Verfahrensschritt 161 eine farbliche Kodierung des Vergleichsergebnisses und/oder mit Verfahrensschritt 162 eine Visualisierung von farblich kodierten Differenzen umfassen. Verfahrensschritt 110, die Bereitstellung von präoperativen MR-Bilddaten, kann optional mit Verfahrensschritt 111 eine Akquisition der präoperativen MR-Bilddaten unter Verwendung einer DIXON-MR-Steuerungssequenz, insbesondere einer VIBE-DIXON-MR-Steuerungssequenz, umfassen.

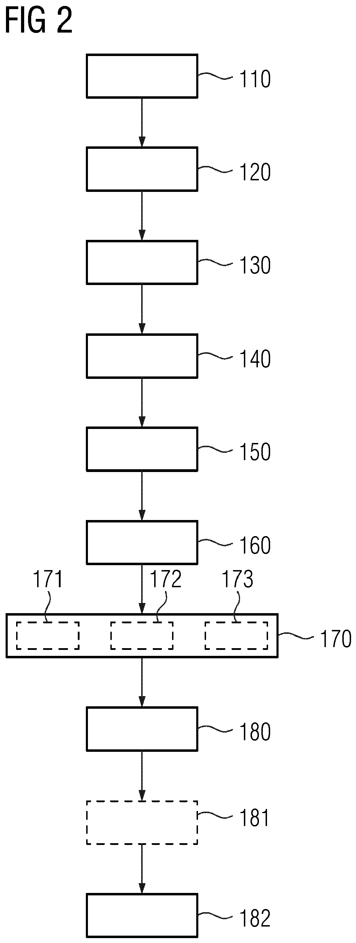

Figur 2 zeigt ein Ablaufdiagramm einer zweiten Ausführungsform eines erfindungsgemäßen Verfahrens. Die zweite Ausführungsform unterscheidet sich von der in Figur 1 dargestellten ersten Ausführungsform typischerweise durch die zusätzlichen Verfahrensschritte 170, 180, 182. Verfahrensschritt 170 umfasst eine Bestimmung zumindest eines Teilbereiches der segmentierten Lipid-MR-Daten basierend auf dem Vergleichsergebnis und/oder der Lipidkarte, welcher Teilbereich einem zu entfernenden Gewebe entspricht. Optional kann hierbei mit Verfahrensschritt 171 eine Auswahl eines Körperbereiches des Untersuchungsobjektes und/oder mit Verfahrensschritt 172 eine Vorgabe einer Menge eines zu entfernenden Gewebes und/oder mit Verfahrensschritt 173 eine Prüfung des Teilbereiches hinsichtlich einer pathologischen und/oder physiologischen Relevanz vorgesehen sein. Die zweite Ausführungsform umfasst mit Verfahrensschritt 180 eine Erzeugung simulierter MR-Bilddaten durch Entfernung des Teilbereiches aus den präoperativen MR-Bilddaten und mit Verfahrensschritt 182 eine perspektivische Visualisierung einer Oberfläche der simulierten MR-Bilddaten. Sofern optional in Verfahrensschritt 181 zusätzlich eine dreidimensionale photographische Aufnahme der Körperoberfläche zumindest eines Ausschnittes des Untersuchungsobjektes erfolgt, kann diese in Verfahrensschritt 182 bei der perspektivischen Visualisierung der Oberfläche der simulierten MR-Bilddaten berücksichtigt werden.

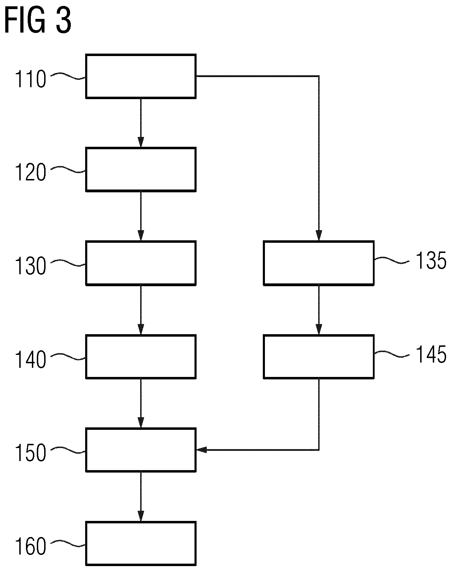

Figur 3 zeigt ein Ablaufdiagramm eines erfindungsgemäßen Verfahrens. Es enthält die zusätzlichen Verfahrensschritte 135, 145. Verfahrensschritt 135 umfasst eine Bestimmung einer Lipiddichtkarte umfassend einen relativen Fettanteil basierend auf den Wasser-MR-Daten und den Lipid-MR-Daten. Verfahrensschritt 145 umfasst eine Bereitstellung einer weiteren Referenzkarte für eine Lipiddichte repräsentativ für einen Idealzustand. In Verfahrensschritt 150, der Ermittlung des Vergleichsergebnisses, wird vorzugsweise eine Differenz zwischen der Lipiddichtkarte und der weiteren Referenzkarte gebildet.

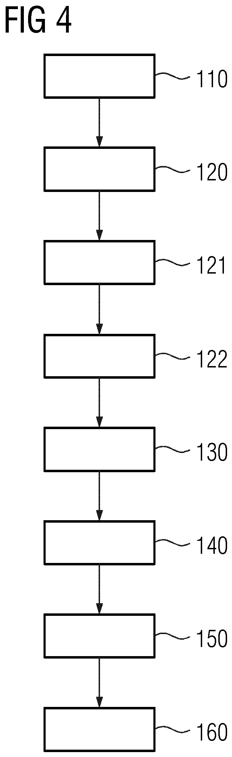

Figur 4 zeigt ein Ablaufdiagramm einer vierten Ausführungsform eines erfindungsgemäßen Verfahrens. Die vierte Ausführungsform unterscheidet sich von der in Figur 1 dargestellten ersten Ausführungsform typischerweise durch die zusätzlichen Verfahrensschritte 121, 122. Verfahrensschritt 121 umfasst eine Subsegmentierung der segmentierten Lipid-MR-Daten gemäß zumindest zwei Lipidklassen und Verfahrensschritt 122 umfasst eine Klassifizierung der subsegmentierten Lipid-MR-Daten gemäß den zumindest zwei Lipidklassen. Vorzugsweise wird in Verfahrensschritt 130 bei Erstellung der Lipidkarte eine erste Lipidklasse der zumindest zwei Lipidklassen ausgeschlossen und/oder die Lipidkarte umfasst eine Information hinsichtlich der Klassifizierung.

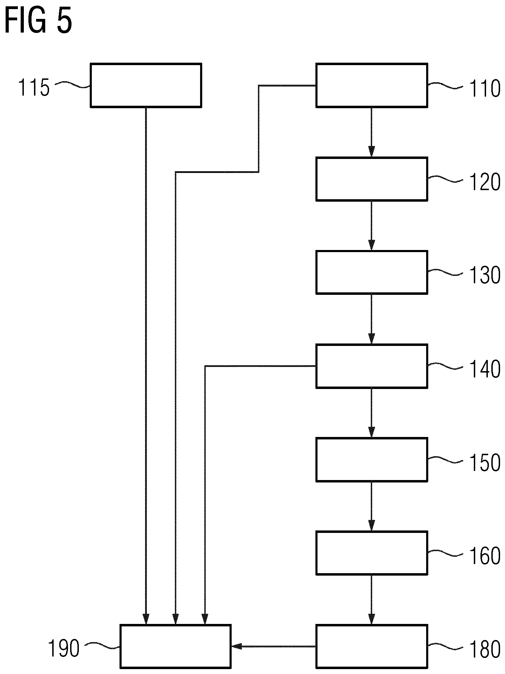

Figur 5 zeigt ein Ablaufdiagramm einer fünften Ausführungsform eines erfindungsgemäßen Verfahrens. Die fünfte Ausführungsform unterscheidet sich von der in Figur 1 dargestellten ersten Ausführungsform typischerweise durch die zusätzlichen Verfahrensschritte 115 und 190. Verfahrensschritt 115 umfasst eine Bereitstellung postoperativer MR-Bilddaten des Untersuchungsbereiches. Verfahrensschritt 190 umfasst einen Vergleich der postoperativen MR-Bilddaten mit den präoperativen MR-Bilddaten und/oder der Referenzkarte und/oder den simulierten MR-Bilddaten.

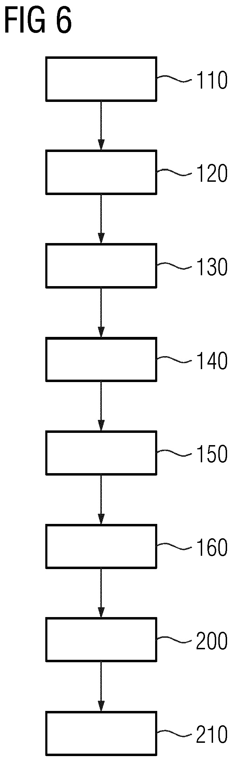

Figur 6 zeigt ein Ablaufdiagramm einer sechsten Ausführungsform eines erfindungsgemäßen Verfahrens. Die sechste Ausführungsform unterscheidet sich von der in Figur 1 dargestellten ersten Ausführungsform typischerweise durch die zusätzlichen Verfahrensschritte 200 und 210.

Verfahrensschritt 200 umfasst ein Erfassen einer Position eines Interventionsgerätes ausgebildet zum Entfernen von Fettgewebe während eines interventionellen Verfahrens zur Entfernung von Fettgewebe. Verfahrensschritt 210 umfasst eine Visualisierung der Position in Überlagerung mit den präoperativen MR-Bilddaten.

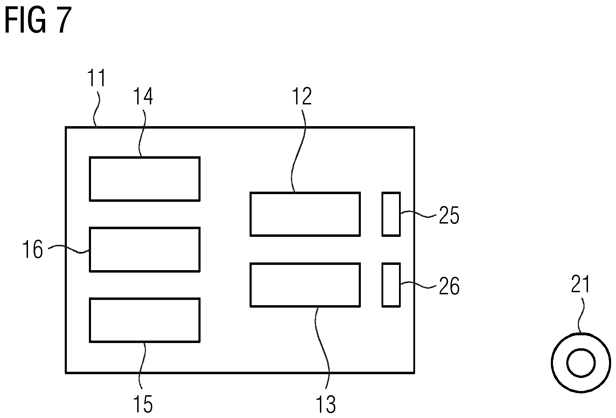

Figur 7 zeigt eine Planungseinheit 11 zur Ausführung eines erfindungsgemäßen Verfahrens in einer schematischen Darstellung. Die Planungseinheit 11 weist hierzu einen Eingang 12, eine Segmentierungseinheit 14, eine Extraktionseinheit 15, eine Differenzierungseinheit 16 und einen Ausgang 13 auf. Die Planungseinheit 11 kann eine nicht näher dargestellte Bestimmungseinheit und/oder Simulationseinheit und/oder Sensoreinheit und/oder Speichereinheit und/oder Recheneinheit umfassen. Die Planungseinheit 11 weist eine Anzeigeeinheit 25 auf. Das Vergleichsergebnis, sowie präoperative MR-Bilddaten und/oder postoperative MR-Bilddaten können auf der Anzeigeeinheit 25, beispielsweise auf zumindest einem Monitor, für einen Benutzer angezeigt werden. Zudem weist die Planungseinheit 11 eine Eingabeeinheit 26 auf, mittels derer von einem Benutzer Informationen eingegeben und/oder ein Körperbereich und/oder ein Teilbereich ausgewählt und/oder eine Menge eines zu entfernenden Gewebes vorgegeben werden können.

Zudem weist die Planungseinheit 11 Computerprogramme und/oder Software auf, die direkt in einem nicht näher dargestellten Speichereinheit der Planungseinheit 11 ladbar sind, mit Programmmitteln, um ein Verfahren zu einer Vorbereitung eines ästhetischen operativen Eingriffes auszuführen, wenn die Computerprogramme und/oder Software in der Planungseinheit 11 ausgeführt werden. Die Planungseinheit 11 weist hierzu einen nicht näher dargestellten Prozessor auf, der zu einer Ausführung der Computerprogramme und/oder Software ausgelegt ist. Alternativ hierzu können die Computerprogramme und/oder Software auch auf einem getrennt von der Planungseinheit 11 ausgebildeten elektronisch lesbaren Datenträger 21 gespeichert sein, wobei ein Datenzugriff von der Planungseinheit 11 auf den elektronisch lesbaren Datenträger 21 über ein Datennetz erfolgen kann.

Die dargestellte Planungseinheit 11 kann selbstverständlich weitere Komponenten umfassen, die Planungseinheiten 11gewöhnlich aufweisen. Eine allgemeine Funktionsweise einer Planungseinheit 11 ist zudem dem Fachmann bekannt, so dass auf eine detaillierte Beschreibung der weiteren Komponenten verzichtet wird.

Ein Verfahren zu einer Vorbereitung eines ästhetischen operativen Eingriffes kann auch in Form eines Computerprogrammprodukts vorliegen, das das Verfahren auf die Planungseinheit 11 implementiert, wenn es auf der Planungseinheit 11 ausgeführt wird. Ebenso kann ein elektronisch lesbarer Datenträger 21 mit darauf gespeicherten elektronisch lesbaren Steuerinformationen vorliegen, welche zumindest ein solches eben beschriebenes Computerprogrammprodukt umfassen und derart ausgestaltet sind, dass sie bei Verwendung des Datenträgers 21 in einer Planungseinheit 11 das beschriebene Verfahren durchführen.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einer Vorbereitung eines ästhetischen operativen Eingriffes hinsichtlich eines Untersuchungsbereiches eines Untersuchungsobjektes umfassend die folgenden Verfahrensschritte:
- Bereitstellung (110) von präoperativen MR-Bilddaten des Untersuchungsbereiches umfassend Lipid-MR-Daten aufweisend einen Lipidkontrast und umfassend Wasser-MR-Daten aufweisend einen Wasserkontrast,
- Segmentierung (120) der Lipid-MR-Daten zur Abgrenzung von Fettgewebe von umgebendem Gewebe mittels einer Segmentierungseinheit,
- Erstellung (130) einer Lipidkarte des Untersuchungsobjektes basierend auf den segmentierten Lipid-MR-Daten mittels einer Extraktionseinheit,
- Bestimmung (135) einer Lipiddichtkarte umfassend einen relativen Fettanteil basierend auf den Wasser-MR-Daten und den Lipid-MR-Daten mittels einer Bestimmungseinheit,
- Bereitstellung (140) einer Referenzkarte für Fettgewebe repräsentativ für einen Idealzustand, wobei der Idealzustand abhängig von individuellen Daten des Untersuchungsobjektes ist,
- Bereitstellung (145) einer weiteren Referenzkarte für eine Lipiddichte repräsentativ für den Idealzustand,
- Ermittlung (150) eines Vergleichsergebnisses umfassend eine Differenz zwischen der Lipidkarte und der Referenzkarte und eine Differenz zwischen der Lipiddichtkarte und der weiteren Referenzkarte mittels einer Differenzierungseinheit,
- Bestimmung zumindest eines Teilbereiches der segmentierten Lipid-MR-Daten basierend auf dem Vergleichsergebnis, welcher Teilbereich einem zu entfernenden Gewebe entspricht, umfassend eine Prüfung des Teilbereiches hinsichtlich einer pathologischen und/oder physiologischen Relevanz mittels der Bestimmungseinheit,
- Bereitstellung des Vergleichsergebnisses.

2. Verfahren nach Anspruch 1,
wobei die Bereitstellung des Vergleichsergebnisses eine farbliche Kodierung des Vergleichsergebnisses und Visualisierung von farblich kodierten Differenzen umfasst.

3. Verfahren nach einem der vorangehenden Ansprüche, zusätzlich umfassend eine Bestimmung zumindest eines Teilbereiches der segmentierten Lipid-MR-Daten basierend auf der Lipidkarte, welcher Teilbereich einem zu entfernenden Gewebe entspricht.

4. Verfahren nach einem der vorangehenden Ansprüche, zusätzlich umfassend eine Erzeugung simulierter MR-Bilddaten durch Entfernung des Teilbereiches aus den präoperativen MR-Bilddaten.

5. Verfahren nach Anspruch 4,
zusätzlich umfassend eine perspektivische Visualisierung einer Oberfläche der simulierten MR-Bilddaten und eine dreidimensionale photographische Aufnahme der Körperoberfläche zumindest eines Ausschnittes des Untersuchungsobjektes, welche bei der perspektivischen Visualisierung der Oberfläche der simulierten MR-Bilddaten berücksichtigt wird.

6. Verfahren nach einem der vorangehenden Ansprüche,
wobei die Bereitstellung der präoperativen MR-Bilddaten deren Akquisition unter Verwendung einer DIXON-MR-Steuerungssequenz, insbesondere einer VIBE-DIXON-MR-Steuerungssequenz, umfasst.

7. Verfahren nach einem der vorangehenden Ansprüche, zusätzlich umfassend eine
- Subsegmentierung der segmentierten Lipid-MR-Daten gemäß zumindest zwei Lipidklassen,
- Klassifizierung der subsegmentierten Lipid-MR-Daten gemäß den zumindest zwei Lipidklassen,
wobei bei Erstellung der Lipidkarte eine erste Lipidklasse der zumindest zwei Lipidklassen ausgeschlossen wird und/oder die Lipidkarte eine Information hinsichtlich der Klassifizierung umfasst.

8. Verfahren nach einem der vorangehenden Ansprüche, zusätzlich umfassend eine Bereitstellung postoperativer MR-Bilddaten des Untersuchungsbereiches und einen Vergleich der postoperativen MR-Bilddaten mit den präoperativen MR-Bilddaten und/oder der Referenzkarte und/oder den simulierten MR-Bilddaten unter der Bedingung, dass nach einem der Ansprüche 4 bis 5 simulierte MR-Bilddaten erzeugt werden.

9. Planungseinheit ausgebildet zur Durchführung eines Verfahrens zu einer Vorbereitung eines ästhetischen operativen Eingriffes hinsichtlich eines Untersuchungsbereiches eines Untersuchungsobjektes umfassend einen Eingang (12), über welchen Eingang
- präoperative MR-Bilddaten des Untersuchungsbereiches umfassend Lipid-MR-Daten aufweisend einen Lipidkontrast und umfassend Wasser-MR-Daten aufweisend einen Wasserkontrast,
- eine Referenzkarte für Fettgewebe repräsentativ für einen Idealzustand, wobei der Idealzustand abhängig von individuellen Daten des Untersuchungsobjektes ist, und
- eine weitere Referenzkarte für eine Lipiddichte repräsentativ für den Idealzustand, bereitgestellt werden können,
eine Segmentierungseinheit (14) ausgebildet zur Segmentierung der Lipid-MR-Daten zur Abgrenzung von Fettgewebe von umgebendem Gewebe,
eine Extraktionseinheit (15) ausgebildet zur Erstellung einer Lipidkarte des Untersuchungsobjektes basierend auf den segmentierten Lipid-MR-Daten,
eine Differenzierungseinheit (16) ausgebildet zur Ermittlung eines Vergleichsergebnisses umfassend eine Differenz zwischen der Lipidkarte und der Referenzkarte und eine Differenz zwischen der Lipiddichtkarte und der weiteren Referenzkarte,
eine Bestimmungseinheit ausgebildet zur Bestimmung zumindest eines Teilbereiches der segmentierten Lipid-MR-Daten basierend auf dem Vergleichsergebnis, welcher Teilbereich einem zu entfernenden Gewebe entspricht, umfassend eine Prüfung des Teilbereiches hinsichtlich einer pathologischen und/oder physiologischen Relevanz,
und einen Ausgang (13) ausgebildet zur Bereitstellung des Vergleichsergebnisses.

10. Planungseinheit nach Anspruch 9,
wobei die Bestimmungseinheit zusätzlich zu
- einem Erfassen einer Position eines Interventionsgerätes ausgebildet zum Entfernen von Fettgewebe während eines interventionellen Verfahrens zur Entfernung von Fettgewebe, und
- einer Visualisierung der Position in Überlagerung mit den präoperativen MR-Bilddaten
ausgebildet ist.

11. Computerprogrammprodukt, welches ein Programm umfasst und direkt in einen Speicher einer programmierbaren Planungseinheit nach Anspruch 9 oder 10 ladbar ist, mit Programmmitteln, um ein Verfahren zu einer Vorbereitung eines ästhetischen operativen Eingriffes nach einem der Ansprüche 1 bis 8 auszuführen, wenn das Programm in der Planungseinheit ausgeführt wird.

12. Elektronisch lesbarer Datenträger, auf dem ein Programm hinterlegt ist, das derart ausgestaltet ist, dass das Programm bei Verwendung des Datenträgers in einer Planungseinheit nach Anspruch 9 oder 10 das Verfahren zu einer Vorbereitung eines ästhetischen operativen Eingriffes nach einem der Ansprüche 1 bis 8 durchführt.

## Claims

1. Method for preparing an aesthetic operative intervention with regard to an examination region of an examination object comprising the following method steps:
- providing (110) preoperative MR image data of the examination region comprising lipid MR data having a lipid contrast and comprising water MR data having a water contrast,
- segmenting (210) the lipid MR data to delineate fat tissue from surrounding tissue by means of a segmentation unit,
- creating (130) a lipid map of the examination object based on the segmented lipid MR data by means of an extraction unit,
- determining (135) a lipid density map comprising a relative fat component based on the water MR data and the lipid MR data by means of a determination unit,
- providing (140) a reference map for fat tissue that is representative of an ideal state, wherein the ideal state is dependent upon individual data of the examination object,
- providing (145) a further reference map for a lipid density that is representative of the ideal state,
- ascertaining (150) a comparison result comprising a difference between the lipid map and the reference map and a difference between the lipid density map and the further reference map by means of a differentiation unit,
- determining at least one subregion of the segmented lipid MR data based on the comparison result, which subregion corresponds to a tissue to be removed, comprising a checking of the subregion with regard to a pathological and/or physiological relevancy by means of a determination unit,
- providing the comparison result.

2. Method according to claim 1,
wherein the providing of the comparison result comprises a colour coding of the comparison result and visualisation of colour-coded differences.

3. Method according to one of the preceding claims, additionally comprising a determining of at least one subregion of the segmented lipid MR data based on the lipid map, which subregion corresponds to a tissue to be removed.

4. Method according to one of the preceding claims, additionally comprising a generating of simulated MR image data by removing the subregion from the preoperative MR image data.

5. Method according to claim 4,
additionally comprising a perspective visualisation of a surface of the simulated MR image data and a three-dimensional photographic recording of the body surface of at least one section of the examination object, which is taken into consideration during the perspective visualisation of the surface of the simulated MR image data.

6. Method according to one of the preceding claims,
wherein the providing of the preoperative MR image data comprises the acquisition thereof with the use of a DIXON MR control sequence, in particular a VIBE DIXON MR control sequence.

7. Method according to one of the preceding claims, additionally comprising
- subsegmenting the segmented lipid MR data according to at least two lipid classes,
- classifying the subsegmented lipid MR data according to the at least two lipid classes,
wherein, when creating the lipid map, a first lipid class of the at least two lipid classes is excluded and/or the lipid map comprises an item of information with regard to the classification.

8. Method according to one of the preceding claims, additionally comprising a providing of postoperative MR image data of the examination region and a comparison of the postoperative MR image data with the preoperative MR image data and/or the reference map and/or the simulated MR image data provided that simulated data is generated according to one of claims 4 to 5.

9. Planning unit embodied to perform a method for preparing an aesthetic operative intervention with regard to an examination region of an examination object comprising an input (12), via which input it is possible to provide
- preoperative MR image data of the examination region comprising lipid MR data having a lipid contrast and comprising water MR data having a water contrast,
- a reference map for fat tissue that is representative of an ideal state, wherein the ideal state is dependent upon individual data of the examination object, and
- a further reference map for a lipid density that is representative of the ideal state,
a segmentation unit (14) embodied for segmenting the lipid MR data to delineate fat tissue from surrounding tissue,
an extraction unit (15) embodied for creating a lipid map of the examination object based on the segmented lipid MR data,
a differentiation unit (16) embodied for ascertaining a comparison result comprising a difference between the lipid map and the reference map and a difference between the lipid density map and the further reference map,
a determination unit embodied for determining at least one subregion of the segmented lipid MR data based on the comparison result, which subregion corresponds to a tissue to be removed, comprising a checking of the subregion with regard to a pathological and/or physiological relevancy,
and an output (13) embodied for providing the comparison result.

10. Planning unit according to claim 9,
wherein the determination unit is additionally provided for
- detecting a position of an intervention device embodied for removing fat tissue during an interventional procedure for removing fat tissue, and
- visualising the position in an overlay with the preoperative MR image data.

11. Computer program product, which comprises a program and can be loaded directly into a memory of a programmable planning unit according to claim 9 or 10, with program means for carrying out a method for preparing an aesthetic operative intervention according to one of claims 1 to 8, when the program is executed in the planning unit.

12. Electronically readable data carrier, on which a program is stored, which is embodied in such a manner that the program performs the method for preparing an aesthetic operative intervention according to one of claims 1 to 8 when the data carrier is used in a planning unit according to claim 9 or 10.

## Revendications

1. Procédé de préparation d'une intervention chirurgicale esthétique concernant une partie à examiner d'un objet à examiner comprenant les stades de procédé suivant :
- mise à disposition (110) de données d'image RM préopératoires de la partie à examiner comprenant des données RM de lipides comportant un contraste de lipides et comprenant des données RM d'eau comportant un contraste d'eau,
- segmentation (120) des données RM de lipides pour la délimitation de tissu graisseux de tissus environnants au moyen d'une unité de segmentation,
- établissement (130) au moyen d'une unité d'extraction d'une carte de lipides de l'objet à examiner sur la base de données RM de lipides segmentées,
- détermination (135) au moyen d'une unité de détermination d'une carte de densité de lipides comprenant une proportion relative de graisse sur la base des données RM d'eau et des données RM de lipides,
- mise à disposition (140) d'une carte de référence du tissu graisseux représentative d'un état idéal, dans lequel l'état idéal dépend de données individuelles de l'objet à examiner,
- mise à disposition (145) d'une autre carte de référence d'une densité de lipides représentative de l'état idéal,
- détermination (150) d'un résultat de comparaison comprenant une différence entre la carte de lipides et la carte de référence et une différence entre la carte de densité de lipides et l'autre carte de référence, au moyen d'une unité de différentiation,
- détermination d'au moins un domaine partiel des données RM de lipides segmentées sur la base du résultat de comparaison, lequel domaine partiel correspond à un tissu à enlever, comprenant un contrôle du domaine partiel en ce qui concerne une pertinence pathologique et/ou physiologique au moyen de l'unité de détermination,
- mise à disposition du résultat de la comparaison.

2. Procédé suivant la revendication 1,
dans lequel la mise à disposition du résultat de la comparaison comprend un codage coloré du résultat de la comparaison et une visualisation de différences codées en couleur.

3. Procédé suivant l'une des revendications précédentes, comprenant en outre une détermination d'au moins un domaine partiel des données RM de lipides segmentées sur la base de la carte de lipides, lequel domaine partiel correspond à un tissu à enlever.

4. Procédé suivant l'une des revendications précédentes, comprenant en outre une production de données d'image RM simulées par enlèvement du domaine partiel des données d'image RM préopératoires.

5. Procédé suivant la revendication 4,
comprenant en outre une visualisation en perspective d'une surface des données d'image RM simulées et un enregistrement photographique en trois dimensions de la surface du corps d'au moins une partie de l'objet à examiner, qui est prise en compte lors de la visualisation en perspective de la surface des données d'image RM simulées.

6. Procédé suivant l'une des revendications précédentes,
dans lequel la mise à disposition des données d'image RM préopératoires comprend leur acquisition en utilisant une séquence de commande RM DIXON, en particulier une séquence de commande RM VIBE-DIXON.

7. Procédé suivant l'une des revendications précédentes, comprenant en outre
- un sous-segmentation suivant au moins deux classes de lipides des données RM de lipides segmentées,
- une classification suivant les au moins deux classes de lipides des données RM de lipides sous-segmentées,
dans lequel, lors de l'établissement de la carte de lipides, on exclut une première classe de lipides des au moins deux classes de lipides et/ou la carte de lipides comprend une information en ce qui concerne la classification.

8. Procédé suivant l'une des revendications précédentes, comprenant en outre une mise à disposition de données d'image RM postopératoire de la partie à examiner et une comparaison des données d'image RM postopératoires aux données d'image RM préopératoires et/ou à la carte de référence et/ou aux données d'image RM simulées à la condition qu'aient été produites des données d'image RM simulées suivant l'une des revendications 4 à 5.

9. Unité de planification constituée pour effectuer un procédé de prétraitement d'une intervention chirurgicale esthétique en ce qui concerne une partie à examiner d'un objet à examiner comprenant une entrée (12), entrée par laquelle peuvent être mises à disposition :
- des données d'image RM préopératoires de la partie à examiner comprenant des données RM de lipides comportant un contraste de lipides et comprenant des données RM d'eau comportant un contraste d'eau,
- une carte de référence du tissu graisseux représentative d'un état idéal, dans laquelle l'état idéal dépend de données individuelles de l'objet à examiner, et
- une autre carte de référence d'une densité de lipides représentative de l'état idéal,
une unité (14) de segmentation constituée pour segmenter les données RM de lipides afin de délimiter des tissus graisseux des tissus environnants,
une unité (15) d'extraction constituée pour l'établissement d'une carte de lipides de l'objet à examiner sur la base des données RM de lipides segmentées,
une unité (16) de différenciation constituée pour la détermination d'un résultat de comparaison comprenant une différence entre la carte de lipides et la carte de référence et une différence entre la carte de densité de lipides et l'autre carte de référence,
une unité de détermination constituée pour la détermination d'au moins un domaine partiel des données RM de lipides segmentées sur la base du résultat de la comparaison, lequel domaine partiel correspond à un tissu à enlever, comprenant un contrôle du domaine partiel en ce qui concerne une pertinence pathologique et/ou physiologique,
et une sortie (13) constituée pour la mise à disposition du résultat de la comparaison.

10. Unité de planification suivant la revendication 9,
dans laquelle l'unité de détermination est constituée en outre
- pour une détection d'une position d'un appareil d'intervention pour enlever du tissu graisseux pendant un procédé chirurgical d'enlèvement de tissu graisseux, et
- une visualisation de la position en superposition avec les données d'image RM préopératoires.

11. Produit de programme d'ordinateur, qui comprend un programme et qui peut être chargé directement dans la mémoire d'une unité de planification programmable suivant la revendication 9 ou 10, comprenant des moyens de programme pour exécuter un procédé de préparation d'une intervention chirurgicale esthétique suivant l'une des revendications 1 à 8, lorsque le programme est exécuté dans l'unité de planification.

12. Support de données, déchiffrable électroniquement, sur lequel est mis en mémoire un programme qui est conformé de manière à ce que le programme exécute, lors de l'utilisation du support de données dans une unité de planification suivant la revendication 9 ou 10, le procédé de préparation d'une intervention chirurgicale esthétique suivant l'une des revendications 1 à 8.
